(19) Europäisches Patentamt
European Patent Office
Office européen des brevets

(11) Veröffentlichungsnummer: **0 270 106**
**A1**

(12) # EUROPÄISCHE PATENTANMELDUNG

(21) Anmeldenummer: 87117867.9

(22) Anmeldetag: 03.12.87

(51) Int. Cl.4: **C07C 85/11** , C07C 87/52 , C07C 87/62

(30) Priorität: 04.12.86 DE 3641500

(43) Veröffentlichungstag der Anmeldung: 08.06.88 Patentblatt 88/23

(84) Benannte Vertragsstaaten: BE CH DE FR GB IT LI NL

(71) Anmelder: BASF Aktiengesellschaft
Carl-Bosch-Strasse 38
D-6700 Ludwigshafen(DE)

(72) Erfinder: Dockner, Toni, Dr.
Grossgasse 6
D-6701 Meckenheim(DE)
Erfinder: Sauerwald, Manfred, Dr.
Gebhardtstrasse 16
D-6701 Roedersheim-Gronau(DE)

(54) **Verfahren zur Reduktion von aromatischen Verbindungen mit NO-Doppelbindungen.**

(57) Verfahren zur Reduktion von aromatischen Verbindungen mit NO-Doppelbindungen durch Erhitzen dieser Verbindungen in Gegenwart eines Wasserstoffdonators, indem man die Reduktion bei Temperaturen von 150 bis 500°C in einem hochsiedenden Mineralöl, dessen Siedepunkt höher als die Reaktionstemperatur liegt, vornimmt, die Reaktionsprodukte in an sich bekannter Weise isoliert und gegebenenfalls das Mineralöl bei Anreicherung mit Nebenprodukten erneuert.

EP 0 270 106 A1

## Verfahren zur Reduktion von aromatischen Verbindungen mit NO-Doppelbindungen

Die vorliegende Erfindung betrifft ein Verfahren zur Reduktion von aromatischen Verbindungen mit NO-Doppelbindungen durch Erhitzen dieser Verbindungen in Gegenwart eines Wasserstoffdonators.

Von den zahlreichen in der Literatur beschriebenen Methoden zur Reduktion von NO-Doppelbindungen verläuft die katalytische Hydrierung an Metallkatalysatoren wie Platin, Palladium oder Raney-Nickel besonders glatt. Anstelle von molekularem Wasserstoff können auch leicht dehydrierbare Verbindungen, wie z.B. Cyclohexen, eingesetzt werden, wobei ebenfalls Edelmetallkatalysatoren erforderlich sind (Houben-Weyl, Methoden der organischen Chemie, Bd. IV/1c, 1980, S. 76 bis 77). Bei Verwendung von Hydrazin als Wasserstoffdonator sind z.B. mit Salpetersäure oxidierte Kohlen als Katalysator geeignet (J. Appl. Chem. USSR 46, 1235, 1973). Nachteile dieser Verfahren sind die Notwendigkeit von Metall-, insbesondere Edelmetallkatalysatoren, die häufig aus ökologischen Gründen mit hohen Kosten zurückgeführt werden müssen sowie bei den Transferhydrierungen der Einsatz relativ teurer Wasserstoffdonatoren.

In Synthesis 1978, S. 23 bis 24 ist ein Verfahren zur Reduktion aromatischer Nitroverbindungen beschrieben, wobei flüssige Paraffine, Leichtbenzin oder Cyclohexan als Wasserstoffdonatoren fungieren. Diese Reduktionen verlaufen bei Temperaturen von 360 bis 400°C mit Umsätzen von 66 bis 100 % und Ausbeuten im Bereich von 28 bis 95 %. Bei den verwendeten Wasserstoffdonatoren handelt es sich ausschließlich um saubere, rein paraffinische Substanzen. In Analogie zur Transferhydrierung mit Hydrazin wird ein pericyclischer Mechanismus postuliert, wobei die Paraffine in Olefine übergehen. Aufgrund dieser Verfahrensbeschreibung war anzunehmen, daß andere Kohlenwasserstoffe, wie z.B. Olefine bzw. Aromaten, schlechter oder gar nicht reagieren.

Der Erfindung lag die Aufgabe zugrunde, ein Verfahren zur Reduktion aromatischer Verbindungen mit NO-Doppelbindungen zu finden, das sich durch besondere Wirtschaftlichkeit auszeichnet und hohe Umsätze bei guter Selektivität liefert sowie insbesondere zur Anwendung im großtechnischen Betrieb geeignet ist.

Demgemäß wurde ein Verfahren zur Reduktion von aromatischen Verbindungen mit NO-Doppelbindungen durch Erhitzen dieser Verbindungen in Gegenwart eines Wasserstoffdonators gefunden, das sich dadurch auszeichnet, daß man die Reduktion bei Temperaturen von 150 bis 500°C in einem hochsiedenden Mineralöl, dessen Siedepunkt höher als die Reaktionstemperatur liegt, vornimmt, die Reaktionsprodukte in an sich bekannter Weise isoliert und gegebenenfalls das Mineralöl bei Anreicherung mit Nebenprodukten erneuert.

Der Erfolg des Verfahrens ist überraschend, da nicht zu erwarten war, daß hochsiedende Mineralöle, die hauptsächlich aus Aromaten und Olefinen bestehen und nur einen geringen Paraffinanteil aufweisen, die zudem noch mit einer Vielzahl unbekannter Verbindungen, die z.B. Schwefel, Eisen, Nickel oder Vanadium enthalten, verunreinigt sind, als Reduktionsmittel geeignet sind. Durch Verwendung völlig uneinheitlicher Mineralölfraktionen bzw. Mineralölrückstände können z.B. aromatische Nitro-und/oder Nitrosoverbindungen in unerwartet hohen Ausbeuten zu den entsprechenden Aminen reduziert werden.

Die Umsetzung läßt sich durch folgende Reaktionsgleichung beschreiben:

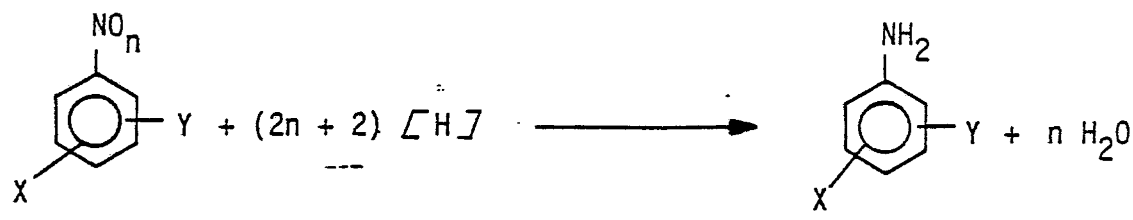

X, Y = H, Alkyl, Aryl, Acyl
F, Cl, Br, CN, OH, $NH_2$

n = 1,2

Der für die Umsetzung erforderliche Wasserstoff entstammt den Kohlenwasserstoffen des Mineralöls, die dabei in wasserstoffarme Derivate bis hin zum Kohlenstoff übergehen. Als hochsiedendes Mineralöl können vorteilhaft billige Raffinerieprodukte wie Vakuumgasöl, schweres Heizöl oder Vakuumrückstand Verwendung finden. Die Siedepunkte dieser Öle liegen über der jeweiligen Reaktionstemperatur, die in der

Regel 150 bis 500°C, insbesondere 150 bis 350°C, vorzugsweise 200 bis 400°C und besonders bevorzugt 250 bis 350°C beträgt.

Als Ausgangsstoffe können vorteilhaft aromatische Nitro-und/oder Nitrosoverbindungen vewendet werden. Als aromatische Grundkörper dienen z.B. Benzol, kondensierte Aromaten wie Naphthalin, Anthracen oder Phenanthren oder Heteroaromaten mit einem oder mehreren, insbesondere 1 bis 2 Heteroatomen wie Sauerstoff, Schwefel und insbesondere Stickstoff, z.B. Pyridin, Chinolin, Pyrimidin.

Neben der oder den zu reduzierenden Nitro-oder Nitrosogruppen können weitere unter den Reaktionsbedingungen inerte Substituenten am Aromaten vorhanden sein. Vorzugsweise werden Aromaten mit 1 oder 2 Nitro-oder Nitrosogruppen verwendet, die inerte Substitutenten wie Alkyl-oder Alkoxygruppen, z.B. $C_1$ - bis $C_8$ -Alkyl-oder Alkoxy-, Aryl-, z.B. Phenyl-, Aralkyl-, z.B. Benzyl-, Acyl-, Cyano-, Hydroxy-oder Aminogruppen oder Halogensubstituenten wie Fluor, Chlor oder Brom tragen können.

Zweckmäßig wird die Umsetzung so ausgeführt, daß die zu reduzierende Verbindung fest, flüssig oder gasförmig, gegebenenfalls zusammen mit einem Inertgas wie z.B. Stickstoff, unter der Oberfläche des auf Reaktionstemperatur erhitzten Mineralöls in den Reaktor eingebracht wird. Pro Mol Ausgangsstoff können vorteilhaft 10 bis 2 000 g, insbesondere 100 bis 1 000 g Öl verwendet werden. Vorteilhaft sollte der größte Teil der reagierenden Kohlenwasserstoffe flüssig vorliegen. Je nach Reaktionstemperatur und Siedepunkt verlassen die Reaktionsprodukte unmittelbar nach der Umsetzung das Reaktionsgefäß und werden durch Abkühlung kondensiert, wonach sie nach bekannten Methoden, z.B. destillativ, aufgearbeitet werden. Produkte mit höherem Siedepunkt als die Reaktionstemperatur verbleiben in dem Reaktionsgemisch und werden nach der Umsetzung, in an sich bekannter Weise, beispielsweise durch Destillation oder Extraktion, isoliert. Das rückstandshaltige Öl kann teilweise ausgeschleust und durch Frischöl ergänzt werden.

Das neue Verfahren kann diskontinuierlich oder vorzugsweise kontinuierlich bei Normaldruck, erhöhtem oder vermindertem Druck nach den dafür üblichen Techniken z.B. in Reaktoren wie Rührbehältern oder zylindrischen Reaktoren mit Umlauf durchgeführt werden. Bei der kontinuierlichen Arbeitsweise kann es vorteilhaft sein, das Mineralöl kontinuierlich zuzuführen und abzuziehen, beispielsweise um gegebenenfalls in geringem Umfang entstandene Nebenprodukte wie Polymere, Crack-oder höhersiedende Nebenprodukkte zusammen mit dem Mineralöl aus dem Reaktor auszuschleusen. Eine Aufarbeitung und Rückführung des abgezogenen Mineralöls ist in der Regel nicht wirtschaftlich, da das Mineralöl in der Regel wohlfeil zur Verfügung steht. Man wird daher zweckmäßig das mit Nebenprodukten angereicherte Mineralöl einer Verfeuerung und dem Reaktor frisches Mineralöl zuführen.

Das erfindungsgemäße Verfahren macht es auf eine technisch einfache, billige Art möglich, aromatische Nitro-und/oder Nitrosoverbindungen zu Aminen zu reduzieren. Ein besonderer Vorteil des Verfahrens liegt in dem außerordentlich niedrigen Preis der als Wasserstoffdonatoren verwendeten Mineralöle. Vorteilhaft ist ferner, daß die Hydrierung ohne den Umweg über molekularen Wasserstoff verläuft und damit keine Abtrennung oder Rückgewinnung von Metallen erforderlich ist.

Die folgenden Beispiele sollen das erfindungsgemäße Verfahren verdeutlichen aber keineswegs einschränken.

Beispiel 1

Reduktion von 4-Chlor-2-nitrotoluol zu 4-Chlor-2-aminotoluol

500 g Vakuumrückstand wurden auf 350°C erhitzt und stüdlich 52 g 4-Chlor-2-nitrotoluol (bei 70°C aufgeschmolzen) zusammen mit 30 1 Stickstoff unter der Oberfläche des gerührten Öls zugeführt. Die den Reaktionskolben verlassenden Dämpfe wurden abgekühlt und kondensiert. Nach einer Versuchsdauer von zwei Stunden wurde das Kondensat zwecks Abtrennung von ausgestripptem Öl mit Methanol versetzt, nach Phasentrennung die Ölphase nochmals mit Methanol extrahiert und die vereinigten Extrakte gaschromatographisch untersucht. Man erhielt neben 7,4 g nicht umgesetztem Ausgangsmaterial 76,9 g 4-Chlor-2-aminotoluol. Dies entspricht einem Umsatz von 92,9 % und einer Selektivität von 96,5 %.

Beispiel 2

Reduktion von 2-Chlor-6-nitrotoluol zu 2-Chlor-6-aminotoluol

a) Wie in Beispiel 1 beschrieben, wurden stündlich 52 g 2-Chlor--6-nitrotoluol (bei 70°C aufgeschmolzen) umgesetzt und nach einer Versuchdauer von zwei Stunden das angefallene Kondensat analog aufgearbeitet und untersucht. Man erhielt neben 8,1 g nicht umgesetztem Ausgangsmaterial 73,5 g 2-Chlor-6-aminotoluol. Dies entspricht einem Umsatz von 92,2 % und einer Selektivität von 92,9 %.

b) Führte man die in a) beschriebene Umsetzung in 500 g Vakuumgasöl bei 300°C durch, so erhielt man neben 14,5 g nicht umgesetztem Ausgangsmaterial 65,2 g 2-Chlor-6-aminotoluol. Diese entspricht einem Umsatz von 86,1 % und einer Selektivität von 88,2 %.

Beispiel 3

Reduktion von Nitrobenzol zu Anilin

Entsprechend Beispiel 1 wurden stündlich 37 g Nitrobenzol zusammen mit 30 1 Stickstoff bei 350°C in Vakuumrückstand eingeleitet und die Reaktionsprodukte analog aufgearbeitet und untersucht. Nach einer Versuchsdauer von vier Stunden erhielt man neben 22,9 g nicht umgesetztem Ausgangsmaterial 79,7 g Anilin. Diese entspricht einem Umsatz von 84,5 % und einer Selektivität von 84,3 %.

**Ansprüche**

1. Verfahren zur Reduktion von aromatischen Verbindungen mit NO-Doppelbindungen durch Erhitzen dieser Verbindungen in Gegenwart eines Wasserstoffdonators, dadurch gekennzeichnet, daß man die Reduktion bei Temperaturen von 150 bis 500°C in einem hochsiedenden Mineralöl, dessen Siedepunkt höher als die Reaktionstemperatur liegt, vornimmt, die Reaktionsprodukte in an sich bekannter Weise isoliert und gegebenenfalls das Mineralöl bei Anreicherung mit Nebenprodukten erneuert.

2. Verfahren nach Anspruch 1, dadurch gekennzeichnet, daß man als Mineralöl Vakuumgasöl, schweres Heizöl oder Vakuumrückstand verwendet.

3. Verfahren nach den Ansprüchen 1 und 2, dadurch gekennziechnet, daß man die Reduktion bei einer Temperatur von 150 bis 350°C vornimmt.

4. Verfahren nach den Ansprüchen 1 bis 3, dadurch gekennzeichnet, daß man aromatische Nitro-oder Nitrosoverbindungen zu den entsprechenden Aminen reduziert.

5. Verfahren nach den Ansprüchen 1 bis 4, dadurch gekennzeichnet, daß man die Reduktion kontinuierlich durchführt.

6. Verfahren nach den Ansprüchen 1 bis 5, dadurch gekennzeichnet, daß man die Reaktionsprodukte gasförmig aus dem Reaktionsgemisch abtrennt und anschließend kondensiert.

7. Verfahren nach den Ansprüchen 1 bis 6, dadurch gekennzeichnet, daß man mit Nebenprodukten angereicherte Mineralöle abtrennt und einer Verfeuerung zuführt.

Europäisches Patentamt

# EUROPÄISCHER RECHERCHENBERICHT

Nummer der Anmeldung

EP 87117867.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | SYNTHESIS, 1978, Stuttgart<br>L.BINDIN et al. "A simple method for reducing aromatic nitro-compounds, azo-compounds, and related systems" Seiten 23,24.<br>* Seite 23, rechte Spalte, Zeile 13, Tabelle; Seite 24, linke Spalte, 3. Absatz *<br>-- | 1,3-6 | C 07 C 85/11<br>C 07 C 87/52<br>C 07 C 87/62 |
| A | DE - A1 - 3 207 475 (BAYER AG)<br>* Zusammenfassung; Beispiel 12 *<br>-- | 1,3-5 | |
| A | GB - A - 705 919 (NATIONAL RESEARCH DEVELOPMENT CORP.)<br>* Beispiele *<br>---- | 1,3,4 | |
| | | | RECHERCHIERTE SACHGEBIETE (Int. Cl.4) |
| | | | C 07 C 85/00<br>C 07 C 87/00 |

Der vorliegende Recherchenbericht wurde für alle Patentansprüche erstellt.

| Recherchenort | Abschlußdatum der Recherche | Prüfer |
|---|---|---|
| WIEN | 02-02-1988 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument